(19) Europäisches Patentamt
European Patent Office
Office européen des brevets

(11) Publication number: **0 013 662**
**B1**

(12) **EUROPEAN PATENT SPECIFICATION**

(45) Date of publication of patent specification: **13.04.83**

(21) Application number: **80810004.4**

(22) Date of filing: **07.01.80**

(51) Int. Cl.³: **C 07 D 499/00,**
A 61 K 31/43 //C07D205/08,
C07F9/65

# (54) 2-Penem compounds, a method for their preparation and pharmaceutical compositions comprising them.

(30) Priority: **10.01.79 US 2471**
**01.08.79 US 62875**
**05.11.79 US 91610**

(43) Date of publication of application:
**23.07.80 Bulletin 80/15**

(45) Publication of the grant of the patent:
**13.04.83 Bulletin 83/15**

(84) Designated Contracting States:
**AT BE CH DE FR IT LU NL SE**

(56) References cited:
**EP - A - 0 002 210**
**EP - A - 0 003 960**
**DE - A - 2 819 655**

***The file contains technical information* submitted after the application was filed and not included in this specification**

(73) Proprietor: **SCHERING CORPORATION**
**2000 Galloping Hill Road**
**Kenilworth, New Jersey 07033 (US)**

(72) Inventor: **McCombie, Stuart Walter**
**159 Pleasant Valley Way**
**West Orange, New Jersey 07052 (US)**

(74) Representative: **Antony, Fritz, Dr. et al,**
**P.O. Box 601 Winkelriedstrasse 35**
**CH-6002 Lucerne (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 013 662 B1**

Courier Press, Leamington Spa, England

# 2-Penem compounds, a method for their preparation and pharmaceutical compositions comprising them

The present invention relates to 2-penem compounds of the formula

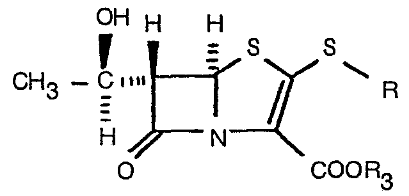

in which R is a $C_1$—$C_6$ alkyl group, and $R_3$ is an alkali metal cation, or a phthalidyl or pivaloyloxymethyl group; or a mixture thereof with its enantiomer.

The $C_1$—$C_6$ alkyl groups referred to above are exemplified by methyl, ethyl, propyl, butyl, pentyl, hexyl, and the corresponding branched-chain isomers thereof.

The alkali metal cations referred to above preferably are potassium and sodium but may also be lithium, rubidium or cesium.

With respect to those chiral centres in the penem nucleus itself, the configurations at the 5 and 6 positions are of the absolute stereochemistry R and S, respectively. The two hydrogen atoms attached to the 5 and 6 carbon atoms are thus *trans* to one another. The C—8 carbon atom has the 8R stereochemistry, the compounds thus being designated 5R, 6S, 8R.

Numerous compounds of the penem class are disclosed in European Patent Applications Publication Nos. —A—0 002 210 (Merck) and A—0 003 960 (Ciba Geigy), both of which are intermediate documents within the meaning of Article 54 paragraph 3, EPC in relation to the present Application. European Application Publication No. A—0 003 960 mentions *inter alia* the compound 6-(1-hydroxyethyl)-2-ethylthio-2-penem-3-carboxylic acid and includes a reference to such a compound in racemic or optically active form and its salts, but does not specifically mention the isomeric form (5R, 6S, 8R) which is an essential characterising feature of the present invention.

European Application Publication No. A—0 002 210 includes various Examples which describe the preparation of compounds defined in terms embracing the compounds of the present invention but, again, without mentioning the particular stereoisomeric form 5R, 6S, 8R. It is noteworthy that two published works, namely Tetrahedron Letters Vol. 21 pp. 619—620 and Vol. 22, No. 36 pp 3489—3492 indicate that the process described in the relevant Examples of EP—A—0 002 210 is inoperable in that the product is an "isopenem" and not a "penem".

Various compounds of the penem class are also disclosed in DE—A—2 819 655 (Ciba Geigy). These penems are, however, unsubstituted in the 6-positions of the penem nucleus.

The compounds of the present invention may be prepared as their racemic mixtures, *e.g.,* the 5R, 6S, 8R compound is produced with its enantiomer (mirror image), *i.e.,* a 5S, 6R, 8S compound, in equal amounts when the starting compound is a racemic mixture. The two enantiomers may be separated by conventional means, *e.g.,* by fractional crystallizations of optically active salt forms, *e.g.,* the salts derived from optically active amino compounds, *e.g.,* (—)-brucine, or (+)- and (—)-ephedrine.

Alternatively, the 5R, 6S, 8R compounds may be produced in their pure enantiomeric form by utilizing optically active starting materials in the synthetic procedure.

The designations of absolute spatial configuration are based on X-ray crystal analysis.

The compounds of this invention possess antibacterial activity of both the gram-positive and gram-negative type. Thus, when tested in standardized microbiological assays, the compounds of this invention are active against such gram-positive organisms as *Staphylococcus epidermidis,* and *Bacillus subtilis,* and such gram-negative organisms as *E. coli* and *Salmonella* at test levels of 0.1 to 100 $\mu$g/ml. Additionally, they show activity against such organisms in the presence of $\beta$-lactamase indicating a resistance to these enzymes and are inhibitors of beta-lactamases. For instance, potassium (5R, 6S, 8R)-6-(1-hydroxyethyl)-2-ethylthiopenem-3-carboxylate is active against *Staphylococcus* 76070103 at a test level of less than 0.06 $\mu$g/ml and against *E. coli* JR66 at a test level of 0.5 $\mu$g/ml. When tested against *B. subtilis* 1119601 (a beta-lactamase-containing organism), this compound exhibits activity at 0.06 $\mu$g/ml.

Thus, the present invention includes within its scope pharmaceutical compositions comprising an antibacterially effective amounts of a penem of formula I (specifically the compound mentioned in the preceding paragraph or a mixture thereof with its enantiomer) together with a compatible, pharmaceutically acceptable carrier or excipient, and dosage forms, specifically for oral use.

The dosage administered of the penems of this invention is dependent upon the age and weight of the animal species being treated, and the type and severity of bacterial infection being prevented or reduced. Typically, the dosage administered per day will be in the range of 100—5000 mg, with 500—1000 mg being preferred.

For oral administration, the compounds of this invention may be formulated in the form of tablets, capsules, elixirs or the like. Likewise, they may be admixed with animal feed.

The compounds of this invention are prepared by cyclising a compound of the formula

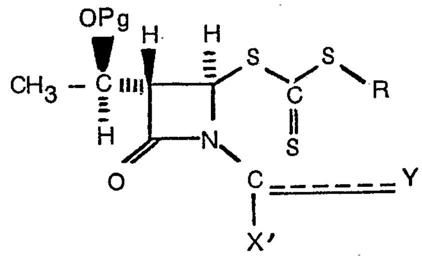

in which X′ is a protected carboxyl group, Pg is a hydroxy protecting group and Y is a phosphonio group being double bonded to the adjacent carbon atom or a phosphonato group with a single bond to the adjacent carbon atom the negative charge of which is compensated by the presence of a cation, or a mixture of stereoisomers containing the same; separating a mixture of diastereoisomers, if a mixture containing diastereoisomers was subjected to cyclisation, before or after removing the protecting groups; and isolating the compound of formula I (in which $R_3$ may be H) or a mixture thereof with its enantiomer as an alkali metal salt, or converting the cyclisation product to the phthalidyl or pivaloyloxymethyl ester or a mixture thereof with its respective enantiomer; if desired the process including the step of separating a mixture of enantiomers if a mixture containing an enantiomer of the compound of formula II was subjected to cyclisation.

The cyclization is generally conducted at a temperature between 30 to 160°C and preferably at reflux temperatures in an organic solvent such as benzene, toluene or xylene under an inert atmosphere, *e.g.,* nitrogen or argon. Reaction times generally vary from 12 to 48 hours.

The group Y in the starting material of formula II is a phosphonio group customary for a Wittig reaction, especially a triaryl-, *e.g.* triphenyl- or tri-*p*-methoxyphenyl-, or tri-lower alkyl, *e.g.* tributylphosphonio, or a phosphonato group, *e.g.* diphenylphosphonato or dimethoxyphosphonato.

The conventional carboxy protecting groups, *e.g.* benzyl, *p*-nitrobenzyl and benzhydryl, can be removed by hydrogenation. Certain hydroxy protecting groups such as trichloroethoxycarbonyl may be removed prior to the carboxy protecting group by deprotection via zinc/acetic acid in a suitable aprotic solvent such as tetrahydrofuran. Most preferably, however, the allyl protecting group will be utilized as carboxy protecting group. This group is most preferably removed by utilizing a suitable aprotic solvent, such as tetrahydrofuran, diethyl ether or methylene chloride, with potassium or sodium 2-ethylhexanoate or 2-ethylhexanoic acid and a mixture of a palladium compound and triphenyl phosphine as the catalyst. This deprotection method is particularly suitable for the sensitive betalactam carboxylates of this invention. Use of the potassium or sodium 2-ethylhexanoate provides the corresponding salt, while use of 2-ethylhexanoic acid affords the free carboxy, or hydroxy group.

The starting materials of formula II are preparable by a reaction sequence starting with a compound of the formula

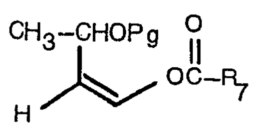

(IV)

wherein

$R_7$ is phenyl or an alkyl group containing 1—6 carbon atoms. The hydroxy substituent must be blocked prior to reaction with a suitable protecting group, designated Pg *e.g.* benzyloxycarbonyl, *p*-nitrobenzyloxycarbonyl, benzhydryloxycarbonyl, allyloxycarbonyl or trichloroethoxycarbonyl. The compound (IV) is reacted with chlorosulfonylisocyanate, followed by a hydrolytic workup, according to procedures disclosed in *Annalen, 1974,* 539, and German Patents 1906401 and 1945549 to provide the intermediate of formula (V)

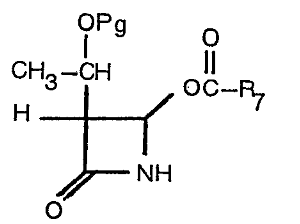

(V)

An alternate method of preparation of the intermediate of formula (V) involves ozone oxidation of a 4-phenyl, 4-methoxyphenyl, or 4-vinylazetidinone appropriately substituted at the 3-position to obtain the 4-carboxylic acid compound. The 4-phenyl and 4-methoxyphenyl compounds are preparable

by the process of *Angew, Chem. Int. Ed. 7,* 172 (1968) while the 4-vinyl compound is preparable from the process of *Canadian J. Chem. 50,* 3196 (1972). The 4-carboxylic acid compound is then treated with lead tetraacetate in an inert solvent to provide the desired intermediate of formula (V).

A further alternative route to intermediates similar to those of formula (V) starts with 4-ethylthioazetidin-2-one (preparable according to the procedures of *Liebigs Ann. Chem., 1974* 539—560. This starting material is treated with a suitable amine-protecting group (for the NH function) to afford the 1-protected-4-ethylthioazetidin-2-one. Preferred protecting groups are those such as *t*-butyldimethylsilyl, triethylsilyl, or tetrahydropyranyl, with *t*-butyldimethylsilyl being particularly preferred. Typically, the reaction is conducted in an organic solvent such as dichloromethane or chloroform in the presence of an acid acceptor. The acid acceptor may be an inorganic or organic base, but organic bases such as triethylamine are generally preferred. Typically, temperatures are from 0°C to room temperature, and typical times range from 5—60 minutes, depending upon the nature of the reactants.

The 1-protected-4-ethylthioazetidin-2-one is treated with a strong base to form an anion at the 3-position which is reacted without isolation with an acetaldehyde to afford the intermediate of the formula (VI):

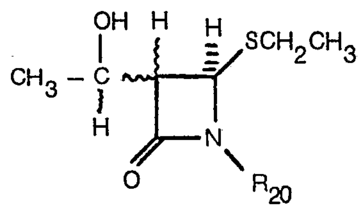

(VI)

wherein

$R_{20}$ is a nitrogen-protecting group. The base utilized to form the anion is one such as lithium diisopropylamine, lithium bis(trimethylsilyl)amide, *n*-butyl lithium or sec-butyl lithium. Generally, an anhydrous aprotic solvent such as tetrahydrofuran or ethyl ether is utilized. Preferred temperatures range from −80°C to −60°C during the production of the anion. After addition of the acetaldehyde the reaction mixture may be allowed to warm to room temperatures. This reaction produces a mixture of 4 isomers which may be utilized further without separation or which may be optionally separated by chromatography at this stage.

The intermediate of formula (VI) is then treated with a suitable hydroxy-blocking reagent to afford the intermediate of the formula (VI′):

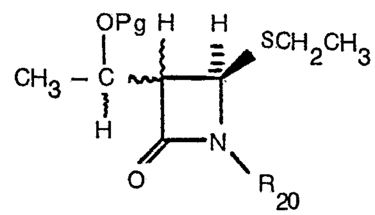

(VI′)

wherein

$R_{20}$ is as hereinbefore defined and Pg is a suitable hydroxy protecting group. Suitable hydroxy protecting groups are those such as 2,2,2-trichloroethoxycarbonyl, 1,1,1-trichloro-2-methyl-2-propoxycarbonyl, *p*-nitrobenzyloxycarbonyl or allyloxycarbonyl, with 2,2,2-trichloroethoxycarbonyl being preferred. Typically, the reaction is conducted in an organic solvent, *e.g.* methylene chloride in the presence of an acid acceptor, *e.g.* triethylamine.

The $R_{20}$-nitrogen-protecting group may then be removed by conventional methods depending upon the exact nature of the $R_{20}$-nitrogen-protecting group utilized. This affords the intermediate of the formula (VII)

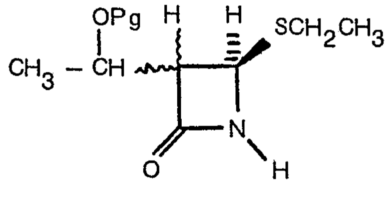

(VII)

wherein

Pg is as hereinbefore defined.

Treatment of the intermediate of formula (V) with a nucleophile of the formula (VIII) or treatment of the intermediate of formula (VII) with chlorine, followed immediately by a nucleophile of the formula (VIII)

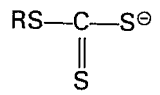

(VIII)

wherein

R is as defined for formula (I) and has suitable amino-, hydroxy and/or carboxy protecting groups, affords the compound of formula (IX)

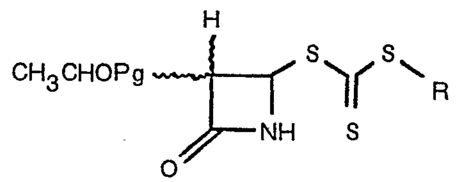

(IX)

A monosubstituted lactam of formula (V) or (VII), will give predominantly the *trans* product in the nucleophilic reaction together with a small amount of the *cis* product. Thus, the compounds of formula (IX) produced will have predominately the following relative stereochemistry

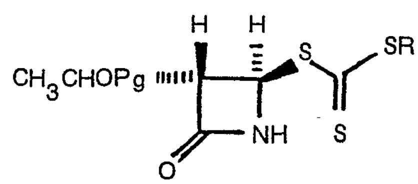

The nucleophile of formula (VIII) is generated *in situ* by reaction of carbon disulfide, the appropriate thiol and a base such as potassium or sodium hydroxide. Generally, the addition of chlorine is at low temperatures (—30 to —10°C), but the subsequent reaction with the nucleophile of formula (VIII) is conducted at slightly higher temperatures, *e.g.* —10 to +10°C. This reaction produces predominantly the two *trans* isomers, *e.g.* the compounds differing in their relative stereochemistry at the asymmetric carbon attached at the 3-position of the azetidin-2-one ring. These two isomers are separated by conventional methods, *e.g.* crystallization and/or chromatography, at this stage of the reaction sequence.

In the foregoing procedure (VI → VI' → VII), if the removal of the $R_{20}$-nitrogen-protecting group and introduction of the protecting group Pg is postponed until after the reaction with chlorine and the nucleophile of formula (VIII), there is produced a product mixture containing predominately the two *cis* isomers of the following formula after appropriate introduction of the Pg-hydroxy-protecting group

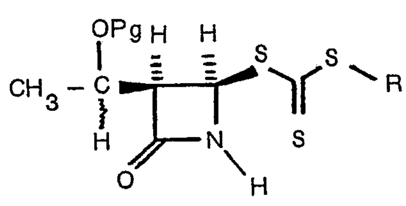

which are then separated by conventional methods, *e.g.* crystallization and/or chromatography, at this stage of the reaction sequence, together with the two *trans* isomers.

The compounds of the above formulae are each produced by the above-described processes in admixture with an equal quantity of their mirror images. Where the pure optically active final products are desired, the intermediates may be resolved by conventional means into their optically active forms. Alternatively, optically active compounds of formula (IX), are preparable by starting from naturally occurring optically active penicillins. One such particularly preferred process comprises:

(a) treatment of an optically active compound of the formula

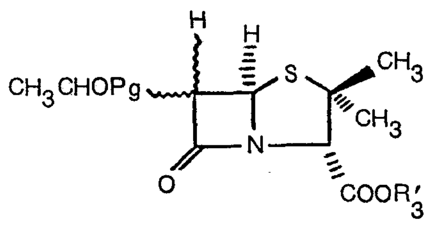

wherein

$R'_3$ is a lower alkyl or aralkyl group, with elemental chlorine, to afford predominately the *trans* intermediate of the formula

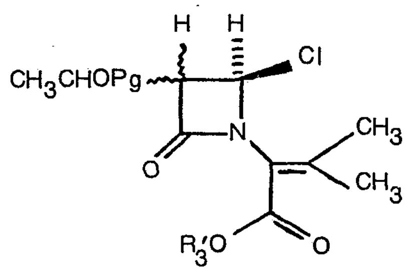

(b) ozonolysis thereof to afford the intermediate of the formula

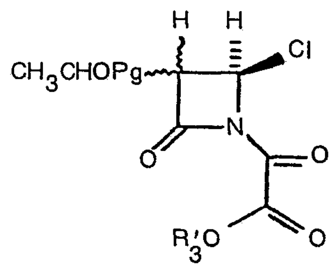

(c) and reaction thereof with a nucleophile of the formula (VIII)

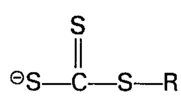 (VIII)

The first step (a) of this process is typically conducted in a suitable organic solvent at temperatures of about −30 to 0°C. Particularly suitable solvents are those such as methylene chloride, chloroform, carbon tetrachloride, toluene and xylene. Preferably, a nitrogen atmosphere is also used. The elemental chlorine is typically added as a solution having concentration of 0.5 to 5 M in a suitable organic solvent, *e.g.* carbon tetrachloride.

The second step (b) of the above described process is typically conducted at low temperatures, *e.g.* about −80 to about −40°C in a non-polar, organic solvent. Most preferably, the same solvent is utilized for this step as for step (a) of the instant process. A particularly suitable solvent is methylene chloride but others, such as chloroform, or xylene, may also be used.

The final step (c) of the above described process may be conducted without isolation and purification of the intermediate. Typically, this reaction is conducted at temperatures of about 0 to 50°C, with room temperatures being most particularly preferred.

The nitrogen of the lactam of formula (IX) is then reacted with an aldehyde X′—CHO to afford the compound of formula (X)

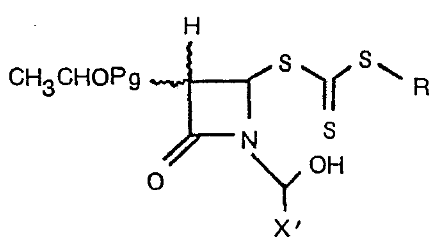 (X)

wherein

X' is protected carboxyl. The carboxyl protecting group may be any suitable group such as *p*-nitrobenzyl, benzyl, allyl or benzhydryl. In a highly preferred embodiment, it will be an allylic group so as to provide the possibility of applying neutral conditions when it is removed at the end of the reaction sequence.

This reaction is usually preferably conducted at reflux temperatures in a non-polar aprotic solvent such as tetrahydrofuran or benzene. Reaction times of 2—10 hours are generally typical.

The compound of formula (X) is then treated with a chlorinating or brominating agent, *e.g.* thionyl chloride, methanesulfonyl chloride, thionyl bromide, or phosphorous tribromide in the presence of an equivalent of an acid acceptor, *e.g.* pyridine or triethylamine, so as to afford replacement of the hydroxy group in position $\alpha$ relative to the ring nitrogen.

Suitable solvents are those such as methylene chloride, tetrahydrofuran or benzene. Temperatures of about 0—20°C and reaction times of 10—60 minutes are generally preferred.

This chloride or bromide is then reacted with a suitable phosphine, *e.g.* tri-*p*-methoxyphenyl phosphine, tributylphosphine or most preferably, triphenylphosphine to afford the compound of the formula (II).

Typically, the reaction is conducted at room temperature in a polar aprotic solvent such as hexamethylphosphoramide or dimethylformamide. Reaction times generally vary from about 12—48 hours.

The transformations of compound (X) to compound (II) are generally according to the procedures described by Woodward, *et. al., Helv. Chim. Acta.,* 55, 408—423 (1972).

Alternatively, in this synthetic procedure, when trimethoxyphosphine or sodium diphenylphosphinate is used in place of *e.g.* triphenylphosphine the corresponding dimethoxyphosphonate or diphenylphosphonate is obtained.

Treatment of this phosphonate with a base such as sodium hydride in a polar solvent (dimethylformamide) results in an unisolated intermediate of the structure

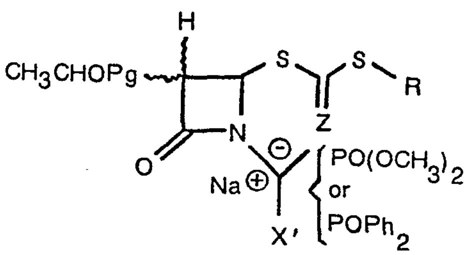

which is also embraced by formula (II).

The compounds of formula (I) wherein $R_3$ is a phthalidyl or pivaloyloxymethyl group are prepared by reaction of the corresponding compound wherein $R_3$ is an alkali metal cation with chlorophthalide or pivaloyloxymethyl chloride in a solvent such as dimethylformamide. Preferably, a catalytic amount of sodium iodide is added.

Compounds preparable by the process of this invention and by following the Preparations and Examples hereinbelow include the following representative compounds of this invention each together with its enantiomer when prepared from racemic starting materials, and alone when prepared from chiral intermediates. The most highly preferred stereochemical isomers are named:

potassium (5R, 6S, 8R)-6-(1-hydroxyethyl)-2-ethylthiopenem-3-carboxylate;
potassium (5R, 6S, 8R)-6-(1-hydroxyethyl)-2-methylthiopenem-3-carboxylate;

The following preparations and examples illustrate the invention. Throughout these preparations and examples, "NMR" denotes nuclear magnetic resonance spectra; "rotation" denotes optical rotation of the compounds in a suitable solvent; "MS" denotes mass spectra; "UV" denotes ultraviolet spectra; and "IR" denotes infrared spectra. Chromatography is performed on silica gel unless otherwise noted.

## Preparation A

(I) 4-Ethylthioazetidin-2-one

Potassium hydroxide (24 g) is dissolved in water (50 ml) and ethanol (300 ml), cooled to 0—5°C and ethanediol (24 g) added, followed by 4-acetoxyazetidin-2-one (43 g). The solution is stirred under nitrogen at room temperature for 20 hours, then added to 10% aqueous sodium chloride solution (1 liter) and extracted four times with 300 ml portions of dichloromethane. The combined extracts are washed twice with saturated sodium chloride, the combined washings back-extracted with an equal volume of dichloromethane, and the combined organic layers dried over anhydrous magnesium sulfate, and evaporated. The residue is dried to constant weight under high vacuum to give the title product as a brown oil, having infrared spectrum $\nu$ max ($CH_2Cl_2$ solution) at 3300, 1765 $cm^{-1}$.

(II) 1-(t-Butyldimethylsilyl)-4-ethylthioazetidin-2-one

A solution of 4-ethylthioazetidin-2-one (43 g) and triethylamine (55 ml) in dichloromethane (300 ml) is stirred at 0—5°C and *t*-butyl(chloro)dimethylsilane (58 g) is added in portions over 5 minutes. The solution is then stirred at room temperature for 0.5 hours. The mixture is washed with 200 ml portions of 0.2 N hydrochloric acid, water and sodium bicarbonate solution, dried and evaporated, and the residue distilled at high vacuum to give a small forerun (e.g., 60—70°C/0.1 mm), followed by the title product, boiling point 110—120°C/0.1 mm pressure as a nearly colorless oil, having infrared spectrum $\nu$ max (film) at 1755 $cm^{-1}$.

(III) 1-(t-Butyldimethylsilyl)-3-(1-hydroxyethyl)-4-ethylthioazetidin-2-one

A solution of lithium di-isopropylamide is prepared by adding 1.6 M butyllithium in hexane (6.7 ml) to diisopropylamine (1.01 g) in tetrahydrofuran (5 ml) at 0°C under argon. The resulting solution is added slowly to a solution of 1-(*t*-butyldimethylsilyl)-4-ethylthioazetidin-2-one (2.45 g) in dry tetrahydrofuran (10 ml) at −70 to −80°C. After 5 minutes, freshly distilled acetaldehyde (1 ml) is added, the mixture warmed to 0°C over 0.5 hour and quenched with acetic acid (1 ml). Dichloromethane (50 ml) is then added, and the solution is washed with water and sodium bicarbonate, dried and evaporated. The residue is dried at high vacuum to give a yellow oil (2.30 g) consisting mainly of the four isomers of the title compound, having infrared spectrum $\nu$ max ($CH_2Cl_2$) at 3300 and 1755 $cm^{-1}$.

Chromatography on silica gel, eluting with 10% ether in dichloromethane gave partial separation, with fractions containing pure samples of the least polar component (a *cis* isomer) and both more polar components (the two *trans* isomers); one of the latter could be crystallized from ether-hexane; melting point 52—53°C.

(IV) 1-(t-Butyldimethylsilyl)-3-(1-trichloroethoxycarbonyloxyethyl)-4-ethylthioazetidin-2-one

A solution of 1-(*t*-butyldimethylsilyl)-3-(1-hydroxyethyl)-4-ethylthioazetidin-2-one (mainly trans isomers; 7.65 g) and pyridine (4.75 ml) in dichloromethane (100 ml) is stirred at 0—5°C, and trichloroethyl chloroformate (6.15 g) is added dropwise. The solution is stirred to room temperature during 1 hour. After washing with 1N sulfuric acid and water, it is dried and evaporated to give the title compound as a pale yellow oil (11.6 g) which can be used without further purification in the next step. A sample partially solidified at −20°C, and two recrystallizations from hexane gave a pure isomer, melting point 92—93°C, having infrared spectrum $\nu$ max ($CH_2Cl_2$) at 1760, 1745 $cm^{-1}$.

(V) 3-(1-Trichloroethoxycarbonyloxyethyl)-4-ethylthioazetidin-2-one

1-(*t*-butyldimethylsilyl)-3-(1-trichloroethoxycarbonyloxyethyl)-4-ethylthioazetidin-2-one (11.55 g) in tetrahydrofuran (160 ml) is stirred with water (20 ml) and concentrated hydrochloric acid (20 ml) for 2.5 hours at room temperature, then isolated by addition of dichloromethane (250 ml), followed by washing with 10% aqueous sodium chloride (2 × 150 ml). The organic phase is then dried and evaporated to the title compound as a yellow oil (6.5 g), having infrared spectrum $\nu$ max (film) at 3400, 1770, 1750 $cm^{-1}$.

(VI) Ethyl-[trans-3-(1-Trichloroethoxycarbonyloxyethyl)-2-azetidinon-4-yl]trithiocarbonate

The product of the previous step (6.5 g) is stirred in dichloromethane (100 ml) at −20°C and a 0.96 M solution of chlorine in carbon tetrachloride (19.4 ml) is added. The resulting solution is added to a rapidly stirred trithiocarbonate solution at 0—5°C prepared from ethanethiol (4.2 ml) in ethanol (50 ml) with addition of 1 M aqueous potassium hydroxide (56 ml) followed by carbon disulfide (15 ml). After stirring the mixture at 0—5°C for 15 minutes, excess dichloromethane is added and the solution is washed with water and aqueous sodium bicarbonate, dried and evaporated.

The resulting mixture is chromatographed on silica gel (100 g), initially eluting with 10% dichloromethane in hexane to remove the by-product [$CH_3CH_2S—S—CS—SCH_2CH_3$, (diethyl tetrathiopercarbonate), a yellow nonpolar oil], then dichloromethane to give the title compound as an approximately 1,3:1 isomeric mixture, the major isomer being the less polar on thin layer chromatography in 5% ether:dichloromethane.

The major isomer is crystallized from the mixture using ester/hexane mixtures, affording in 3 crops a product having a melting point of 92—93°C and having infrared spectrum $\nu$ max ($CH_2Cl_2$) at 3350, 1770 and 1745 $cm^{-1}$ (yellow needless). X-ray crystallography shows this isomer to be the 3S, 4R, 5R isomer.

Chromatography of the final mother liquors on silica gel using 3:1 dichloromethane:hexane gives additional fairly pure major isomer and minor isomer, the latter crystallized with some difficulty from ether-hexane as yellow prisms, melting point 64—67°C.

Preparation B

(I) Ethyl-[1-t-butyldimethylsilyl-3-(1-hydroxyethyl)-2-azetidinone-4-yl]trithiocarbonate (isomeric mixture)

1-*t*-butyldimethylsilyl-3-(1-hydroxyethyl)-4-ethylthio-2-azetidinone (15.0 g of an isomeric mixture prepared in step III of Preparation A) is dissolved in dichloromethane (200 ml) and stirred at −20°C during addition of chlorine in carbon tetrachloride (53 ml of 1.05 M solution).

A thiocarbonate solution is prepared from potassium hydroxide (8.9 g) in water (30 ml) and ethanol (300 ml) with addition of ethanethiol (12 ml), then carbon disulfide (40 ml). This solution is stirred at 0—5°C and the above chlorination mixture added. After 0.5 hours at 0—5°C, the mixture is extracted with dichloromethane, washing with water and aqueous sodium bicarbonate, dried ($MgSO_4$) and evaporated.

The resulting oil is dissolved in carbon tetrachloride and chromatographed on 300 g silica gel, eluting rapidly with carbon tetrachloride to remove the by-product ($CH_3CH_2S—S—CS—S—CH_2CH_3$), followed by 20% ether — carbon tetrachloride to give the title compound as an isomeric mixture as a yellow oil (15 g). By PMR spectrum, the ratio of *cis:trans* is determined to be about 2:1.

(II) Ethyl [1 - t - butyldimethylsilyl - 3 - (1 - trichloroethoxycarbonyloxyethyl) - 2 - azetidinon - 4 - yl]trithiocarbonate

Ethyl [1-*t*-butyldimethylsilyl-3-(1-hydroxyethyl)-2-azetidinon-4-yl]trithiocarbonate (15.0 g) and pyridine (4.3 ml) is stirred in dichloromethane (50 ml) at 0—5°C and trichloroethyl chloroformate (7.4 ml) is added dropwise. The mixture is stirred at room temperature for 2 hours, then diluted with dichloromethane, washed with 0.2 N sulfuric acid, water and sodium bicarbonate. After drying over anhydrous magnesium sulfate, it is evaporated to give a mixture. This mixture is then separated by high pressure liquid chromatography (HPLC) on silica gel, using hexane-dichloromethane mixtures as eluting solvent. The first eluted component is a *trans* isomer (as determined by nuclear magnetic resonance), obtained as an oil. On hydrolysis, it affords the desilylated thiocarbonate, melting point 92—93°C, corresponding to the major isomer of the title compound of Preparation A, step (VI). The second eluted component is a *cis* isomer of the title compound of this example obtained as a yellow oil, $\nu$ max (film) 1750 $cm^{-1}$. The third eluted component is the second *cis* isomer of the title compound, obtained as a waxy yellow solid, melting point 80—85°C. The final component is the remaining *trans* isomer of the title compound, obtained as a yellow oil which on hydrolysis gives the *trans* desilylated thiocarbonate, melting point 64—67°C, corresponding to the minor isomer of Preparation A, step (VI).

(III) cis-Ethyl [3-(1-trichloroethoxycarbonyloxyethyl)-2-azetidinone-4-yl]trithiocarbonate (two isomers)

(i) Isomer I

The first eluted *cis*-N-silyl isomer from step (II) is hydrolyzed by stirring in tetrahydrofuran:water:concentrated hydrochloric acid (20:1:1 by volume) at room temperature until thin layer chromatography (tlc) shows the reaction to be complete. The mixture is extracted in ether:water and the organic phase dried and evaporated. The residue is crystallized from ether:hexane to give yellow needles, melting point 108—111°C.

(ii) Isomer II

By an identical process starting with the second eluted *cis*-N-silyl isomer of step (II), the corresponding thiocarbonate is obtained from ether:hexane as yellow needles, melting point 118—120°C.

Preparation C

(3S, 4R, 5R)-Ethyl [3-(1-trichloroethoxycarbonyloxyethyl-2-azetidinone-4-yl]trithiocarbonate

A. To a solution of 100 g 6-$\beta$-aminopenicillanic acid in 1200 ml 2.5 N sulfuric acid is added 150 g sodium bromide. To the stirred solution at 0°C is added simultaneously 40 g sodium nitrite in 150 ml water and 40 ml bromine. The addition is completed in 10 minutes, maintaining the temperature at 0° to 5°C. The mixture is then stirred rapidly for 1 hour, then filtered. The filter cake is washed with water and taken up in 600 ml ethyl acetate. The ethyl acetate solution is washed with water, cold dilute sodium bisulfite solution and then again with water. After drying over anhydrous sodium sulphate, the solvent is removed under vacuum to afford 67 g in 85:15 ratio (by NMR data) of 6,6-dibromopenicillanic acid and 6-$\beta$-bromopenicillanic acid.

IR: 1728 $cm^{-1}$ and 1800 $cm^{-1}$ (chloroform solution)

NMR: $\delta$ = 5.7, 1H, s; $\delta$ = 4.5, 1H, s; $\delta$ = 1.55—1.67, 6H ($CDCL_3$).

B. To a solution of 67 g in 85:15 ratio of 6,6-dibromopenicillanic acid to 6$\beta$-bromopenicillanic acid in 500 ml dimethylformamide at 0°C is added 37.3 g finely powdered potassium carbonate. The solution is stirred 5—10 minutes and 38.3 g methyl iodide is added. The reaction mixture is then stirred for 2 hours allowing the temperature to come to ambient. The reaction is followed by thin layer chromatography eluting with methylene chloride. When complete, the reaction is decanted and the

solvent removed under high vacuum to leave 100 ml of solution. To this is added 600 ml ethyl acetate. The solution is then washed with water, dried over anhydrous sodium sulphate and concentrated under vacuum to afford 63 g crude methyl ester. Subsequently, 48 g of pure methyl 6,6-dibromopenicillanate is isolated from this crude product by high pressure liquid chromatography eluting with methylene chloride.
NMR: $\delta$ = 5.7, 1H, s; $\delta$ = 4.48, 1H, s; $\delta$ = 3.73, 3H, s; $\delta$ = 1.42, 3H, s; $\delta$ = 1.59, 3H, s ($CDCl_3$).

C. To a solution of 13.7 g methyl 6,6-dibromopenicillanate in 250 ml dry tetrahydrofuran at −78°C under nitrogen is added 14.7 ml of 3 M methyl magnesium bromide in ethyl ether. After stirring for 30 minutes at −78°C, 8 g of freshly distilled acetaldehyde is added and stirring continued for 45 minutes. The reaction mixture is warmed to −20°C at which time 50 ml 1 M potassium phosphate monobasic is added and stirring continued for 5 minutes. The reaction mixture is then poured into 1 liter cold ethyl acetate and washed once with 150 ml brine solution and twice with 150 ml water. The ethyl acetate layer is separated, dried over anhydrous sodium sulfate and evaporated under vacuum. The products, methyl 6$\alpha$-bromo-6$\beta$-(1-hydroxyethyl)penicillanate and methyl 6$\beta$-bromo-6$\alpha$-(1-hydroxyethyl)penicillanate, are detected by thin layer chromatography on silica gel eluting with 10% ethyl acetate/chloroform.

D. To a solution of 8.0 g methyl 6-bromo-6-(1-hydroxyethyl)penicillanate in 200 ml 95% ethanol is added 800 mg 10% palladium on calcium carbonate. The solution is shaken under 2 atmospheres hydrogen pressure for 5 hours. Disappearance of starting material is followed by thin layer chromatography eluting with 20% ethyl acetate/chloroform. The catalyst is filtered and 100 ml 1 M potassium phosphate buffer at pH 7 is added. The precipitate formed is filtered and washed with ethanol. The ethanol is removed under vacuum and 200 ml ethyl acetate added. After washing twice with 50 ml water, and drying over anhydrous sodium sulfate, the ethyl acetate is removed under vacuum to afford a crude mixture of methyl 6-(1-hydroxyethyl)penicillanate. Column chromatography of 18 g of said mixture eluting with 20% ethyl acetate affords 6.4 g methyl (5R, 6S, 8R)-6-(1-hydroxyethyl)-penicillanate.
NMR: $\delta$ = 2.4—2.7, 1H, d; $\delta$ = 4.41, 1H, s; $\delta$ = 3.74, 3H, s; $\delta$ = 3.2—3.33, 1H; $\delta$ = 1.25—1.35, 3H, d; $\delta$ = 1.44, 3H, s; $\delta$ = 1.61, 3H, s ($CDCl_3$).

E. To a solution of 6.2 g methyl (5R, 6S, 8R)-6-(1-hydroxyethyl)penicillanate in 60 ml. dry methylene chloride at 0°C under nitrogen is added 3.8 ml pyridine then 3.3 ml $\beta,\beta,\beta$-trichloroethylchloroformate. The reaction is stirred 15 minutes until all starting material is reacted (as determined by thin layer chromatography with 20% ethyl acetate/chloroform). The solution is poured into 250 ml cold methylene chloride and washed twice with cold 10% phosphoric acid solution, once with cold dilute sodium bicarbonate, and then with water. After drying over anhydrous sodium sulfate, the solvent is removed under vacuum to afford 10.0 g methyl (5R, 6S, 8R)-6-(1-trichlorethoxycarbonyloxyethyl)-penicillanate.
NMR: $\delta$ = 5.13—5.16, 1H, d; $\delta$ = 4.78, 2H, s; $\delta$ = 4.43, 1H, s; $\delta$ = 3.76, 3H, s; $\delta$ = 3.38—3.58, 1H; $\delta$ = 1.45—1.63, 9H; ($CDCl_3$).

F. To a solution of 9.1 g methyl (5R, 6S, 8R)-6-(1-trichloroethoxycarbonyloxyethyl)penicillanate in 350 ml distilled methylene chloride at −20°C under nitrogen is added 62.3 ml of 1 M chlorine/carbon tetrachloride solution. The reaction is stirred for 15 minutes at about −20°C (until found to be complete by thin layer chromatography eluting with chloroform). The solution is evaporated under vacuum to afford 10.0 g of product comprising (3S, 4R, 5R)-1-[(2-methyl-1-methoxycarbonyl)prop-1-enyl]-3-(1-trichloroethoxycarbonyloxyethyl)-4-chloroazetidin-2-one.
IR: 1720, 1770—1790 $cm^{-1}$ (chloroform solution)
NMR: $\delta$ = 5.79—5.81, 1H, d; $\delta$ = 4.75, 2H, s; $\delta$ = 3.74, 3H, s; $\delta$ = 2.27, 3H, s; $\delta$ = 2.0, 3H, s; $\delta$ = 1.45—1.54, 3H, d ($CDCl_3$).

G. Through a solution of 7.7 g crude (3S, 4R, 5R)-1-[2-methyl-1-methoxycarbonyl)prop-1-enyl]-3-(1-trichloroethoxycarbonyloxyethyl)-4-chloroazetidin-2-one in 250 ml methylene chloride at about −78°C is passed ozone for 45 minutes. (Disappearance of starting material is followed by thin layer chromatography eluting with chloroform). The reaction is allowed to sit for 1 hour at −78°C with excess ozone. Nitrogen is then bubbled in for 3—5 minutes and then 3 ml dimethylsulfide is added. The solution is allowed to warm to ambient temperature and held for 2 hours. Nitrogen is bubbled through the solution to remove excess dimethylsulfide. Optionally, the solvent may be removed and the residue purified by chromatography to afford (3S, 4R, 5R)-1-(2-methoxy-1,2-dioxoethyl)-3-(1-trichloroethoxycarbonyloxyethyl)-4-chloroazetidin-2-one.
NMR: $\delta$ = 5.97—6.0, 1H, d; $\delta$ = 5.76, 2H, s; $\delta$ = 4.93, 2H, s; J = 1 c/s, $\delta$ = 1.45—1.55, 3H, d.

H. To a solution of 7.8 g potassium hydroxide in 150 ml water and 150 ml ethanol at 0°C is added 15.3 ml ethanethiol. After stirring for 10 minutes 38.5 ml carbon disulfide is added. The solution

turns deep yellow and is stirred an additional 10 minutes. The solution of step G is cooled to 0°C and poured into this solution. The mixture is then stirred 45 minutes allowing to warm to ambient temperature. The reaction is followed by thin layer chromatography eluting with chloroform. When the reaction is complete, 200 ml methylene chloride is added, followed by 20 g citric acid in 200 ml water. The reaction mixture is stirred 5 minutes and then poured into 500 ml methylene chloride. The organic layer is separated, washed first with water, then with cold dilute sodium bicarbonate solution and then again with water. After drying over anhydrous sodium sulfate, the solvent is removed under vacuum. The crude reaction product is chromatographed on coarse silica gel eluting with 20% chloroform/hexane changing to 100% chloroform to afford 6.4 g (3S, 4R, 5R)-ethyl [3-(1-trichloroethoxycarbonyloxyethyl)-2-azetidinone-4-yl]-trithiocarbonate, alternatively named as (3S, 4R, 5R)-[1-trichloroethoxycarbonyloxyethyl]-4-[(ethylthio)-carbonothioylthio]-azetidin-2-one.

Rotation: $[\alpha]_D^{26} = +154.2°$ (0.4% in dioxane)

NMR: $\alpha$ = 5.6—5.63, 1H, $\alpha$ = 5.1—5.3, 1H, m; $\delta$ = 4.76, 2 H, s; $\delta$ = 3.17—3.52, 3H; $\delta$ = 1.22—1.54, 6H; ($CDCl_3$).

Preparation D

Allyl Glyoxylate Hydrate

Lead tetraacetate (70 g) is added in portions over one half hour to a stirred solution of diallyl tartarate (40 g) in ethyl acetate (400 ml). The mixture is then stirred for an additional one half hour, filtered and washed with ethyl acetate. The filtrate is treated with 10 ml water and evaporated at 50°C/100 mm pressure to remove the ethyl acetate. This residue is distilled at about 30 mm pressure. After initial removal of acetic acid, the title product is collected at 70—80°C/30 mm, as a colorless oil.

Example 1

a. *Ethyl [1-(Allyloxycarbonylhydroxymethyl)-3-(1-trichloroethoxycarbonyloxyethyl)-2-azetidinone-4-yl]trithiocarbonate*

(i) Isomer I

A mixture of the major isomer of the product thiocarbonate from Preparation A (2.13 g), allyl glyoxylate hydrate (1.0 g) from Preparation D) and benzene (25 ml) is refluxed under argon with a water collected for 20 hours. The solution is cooled, diluted with dichloromethane (70 ml) and washed with water (2 × 100 ml), dried and evaporated to give a thin layer chromatography — pure product as a yellow oil.

(ii) Isomer II

Repetition of the procedure of the foregoing paragraph a(i), using the minor isomer of Preparation A (1.02 g) and allyl glyoxylate (0.48 g) in 15 ml benzene with 20 hours reflux is prepared the minor isomer product of this example as a yellow oil.

b. *Ethyl [1-(Allyloxycarbonylchloromethyl)-3-(1-trichloroethoxycarbonyloxyethyl)-2-azetidinone-4-yl]-trithiocarbonate*

(i) Isomer I

The Isomer I product of the foregoing paragraph a(i) (2.77 g) in dichloromethane (30 ml) and methanesulfonylchloride (0.87 g) is stirred at 0°C, and triethylamine (0.78 g), added dropwise. After 5 minutes at room temperature, the solution is diluted with dichloromethane, washed with 5% aqueous tartaric acid and sodium bicarbonate, dried and evaporated to give a brown oil, having infrared spectrum $\nu$ max (film) at 1770 and 1750—1735 (broad) $cm^{-1}$.

(ii) Isomer II

The procedure of the foregoing paragraph b(i) is followed using the Isomer II product of paragraph a(ii) (1.30 g) in 20 ml dichloromethane with triethylamine (0.29 g and mesyl chloride (0.33 g). The final solution after workup is filtered through 5 g silica gel, washing with dichloromethane. Evaporation gives the product as a yellow oil (1.0 g), pure by thin layer chromatography with infrared spectrum $\nu$ max (film) 1770, 1755 and 1735 $cm^{-1}$.

c. *Ethyl [1-(Allyloxycarbonyl[triphenylphosphoranyl]methyl)-3-(1-trichloroethoxycarbonyloxyethyl)-2-azetidinone-4-yl]-trithiocarbonate*

(i) Isomer I

The crude product of the foregoing paragraph b(i) (2.6 g) is stirred with triphenylphosphine (2.8 g) in dry dimethylformamide (30 ml) at room temperature for 40 hours, then extracted with ether, washing with three portions of water. The solution is then dried over anhydrous magnesium sulfate and evaporated. The residue is chromatographed on silica gel (150 g), eluting with 1:1 dichloromethane:hexane to remove excess triphenylphosphine, then with pure dichloromethane to give recovered starting material. The title product is eluted with 5—10% ether-dichloromethane, and pure fractions pooled, evaporated and dried at high vacuum to afford the title compound having infrared spectrum $\nu$ max ($CH_2Cl_2$) at 1750, 1730 and 1690 $cm^{-1}$.

(ii) Isomer II

Using 1.08 g of the Isomer II prepared in the foregoing paragraph b(ii) and triphenylphosphine (0.75 g) in dimethylformamide (15 ml) and repeating the procedure of paragraph c(i) with stirring at room temperature for 72 hours affording the desired Isomer II of the title product as a yellow foam.

d. *Allyl trans-6-(1-trichloroethoxycarbonylethyl)-2-ethylthiopenem-3-carboxylate*

(i) Isomer I

A solution of the Isomer I phosphorane prepared as in paragraph c(i), (0.975 g) in dry toluene (20 ml) is refluxed under argon in an oil bath at 120—125°C for 65 hours, then cooled, diluted with 20 ml hexane and applied to approximately 20 g silica gel. Elution with 1:1 dichloromethane:hexane followed by dichloromethane gives Isomer I of the title compound, which is crystallized from dichloromethane:ether:hexane to give fibrous needles (0.255 g), melting point 123—127°C and having infrared spectrum $\nu$ max ($CH_2Cl_2$) at 1795, 1745, 1700 $cm^{-1}$. *Anal.*
Found: C 38.8; H, 3.6; N, 2.9%
$C_{16}H_{18}NO_6S_2Cl_3$ req: C 39.15; H, 3.7; N, 2.8%

(ii) Isomer II

By similar heating of the Isomer II of the phosphorane as prepared in paragraph c(ii) (0.90 g) in toluene (10 ml) for 36 hours and plate chromatography, there is obtained Isomer II of the title product as a yellow oil having infrared spectrum $\nu$ max ($CH_2Cl_2$) at 1790, 1750, 1705 $cm^{-1}$.

e. *Allyl trans-6-(1-hydroxyethyl)-2-ethylthiopenem-3-carboxylate*

(i) Isomer I

Isomer I of paragraph d(i) (0.175 g) is stirred at 25°C for 1 hour with activated zinc dust (0.05 g) in acetic acid (1 ml) and tetrahydrofuran (3 ml). The mixture is diluted with excess dichloromethane and washed with water, aqueous sodium bicarbonate and aqueous sodium chloride, dried and evaporated. The residue is purified on a preparative thin layer chromatography plate eluting with 20% ether-dichloromethane and crystallized from ether-hexane to afford Isomer I of allyl *trans*-6-(1-hydroxyethyl)-2-ethylthiopenem-3-carboxylate, melting point 65—66°C having infrared spectrum $\nu$ max ($CH_2Cl_2$) at 3250, 1790 and 1705 $cm^{-1}$.

(ii) Isomer II

In a manner similar to that of paragraph e(i), 0.15 g of Isomer II of paragraph d(ii) is deprotected in tetrahydrofuran-acetic acid with zinc dust, and chromatographed on a thin layer chromatography plate to obtain Isomer II of allyl *trans*-6-(1-hydroxyethyl)-2-ethylthiopenem-3-carboxylate, which is crystallized from ether-hexane as cream prisms, melting point 86—88°C, having infrared spectrum $\nu$ max ($CH_2Cl_2$) at 3300, 1795 and 1700 $cm^{-1}$.

f. Repetition of the procedure detailed in paragraphs a) to e) utilizing methyl [3-(1-trichloroethoxycarbonyloxyethyl)-(2-azetidinone-4-yl)] trithiocarbonate, and *n*-butyl[3-(1-trichloroethoxycarbonyloxyethyl)-2-azetidinone-4-yl)]-trithiocarbonate, affords the two isomers of allyl *trans*-6-(1-hydroxyethyl)-2-methylthiopenem-3-carboxylate, and of allyl *trans*-6-(1-hydroxyethyl)-2-(*n*-butylthio) penem-3-carboxylate, respectively.

g) *Potassium trans-6-(1-hydroxyethyl)-2-ethylthiopenem-3-carboxylate*

(i) Isomer I

A solution of Isomer I of allyl *trans*-6-(1-hydroxyethyl)-2-ethylthiopenem-3-carboxylate (52 mg) and 0.5 m potassium-2-ethylhexanoate (0.36 ml) in 1.2 ml ethyl acetate and 0.8 ml dichloromethane is stirred with addition of 3 mg triphenylphosphine and 5 mg of tetrakis (triphenylphosphine) palladium-(0), under argon. After a few minutes, precipitation of product occurs and after 30 minutes, excess ethyl acetate is added and the precipitate centrifuged. Washing with ether and drying at high vacuum gives, as a yellowish powder, the title product having infrared spectrum $\nu$ max (nujol) at 3300, 1775 and 1600 $cm^{-1}$. By X-ray crystallography of the starting materials the stereochemistry of this product is designated 5R, 6S, 8S, together with its enantiomer.

(ii) Isomer II

The procedure of the foregoing paragraph is repeated using Isomer II of allyl *trans*-6-(1-hydroxyethyl)-2-ethylthiopenem-3-carboxylate (40 mg), 2 ml of 1:1 ethyl acetate:dichloromethane, 0.26 ml of 0.5 m potassium-2-ethylhexanoate, 3 mg triphenylphosphine and 5 mg palladium complex. After 20 minutes, ether (2 ml) is added gradually and the product centrifuged and dried under high vacuum to give the title product as a cream powder having infrared spectrum $\nu$ max (nujol) at 3400, 1780 and 1605 $cm^{-1}$. By X-ray crystallography of the starting materials the stereochemistry of this product is designated 5R, 6S, 8R, together with its enantiomer.

h. Repetition of the procedure detailed in step g) using the two isomers of allyl *trans*-6-(1-hydroxyethyl)-2-methylthiopenem-3-carboxylate and of allyl *trans*-6-(1-hydroxyethyl)-2-(*n*-butylthio) penem-3-carboxylate affords the two isomers (5R, 6S, 8S and 5R, 6S, 8R) of potassium *trans*-6-(1-hydroxyethyl)-2-methylthiopenem-3-carboxylate, each together with its enantiomer, and the two isomers (5R, 6S, 8S and 5R, 6S, 8R) of potassium *trans*-6-(1-hydroxyethyl-2-(*n*-butylthio) penem-3-carboxylate, each together with its enantiomer, respectively.

Example 2

The two isomers of potassium *trans*-6-(1-hydroxyethyl)-2-ethylthiopenem-3-carboxylate (0.27 g) of Example 1 g are individually added to a stirred mixture of dry dymethylformamide (2 ml), pivaloyloxymethyl chloride (0.17 ml) and sodium iodide (0.15 g). The mixture is stirred in the dark under nitrogen for 5 hours, then added to water and extracted with ether. The extract is washed with water, a dilute solution of sodium thiosulfate, and finally with saturated sodium chloride solution, dried and evaporated to obtain the two isomers (5R, 6S, 8S and 5R, 6S, 8R) of pivaloyloxymethyl *trans*-6-(1-hydroxyethyl)-2-ethylthiopenem-3-carboxylate, each together with its enantiomer.

Example 3

A. To a solution of 7.7 g (3S, 4R, 5R)-3-[1-trichloroethoxycarbonyloxyethyl]-4-[(ethylthio)-carbonothioylthio]azetidin-2-one in 90 ml benzene is added 3.5 g allyl glyoxalate. Under nitrogen this mixture is slowly azeotroped for 24 hours. (The reaction is followed by thin layer chromatography eluting with 10% ethyl ether/methylene chloride). Approximately 2.0 ml additional allylglyoxalate is added and the reaction is azeotroped for 10 additional hours. The reaction is then cooled and 150 ml benzene is added. The resultant solution is washed 5 times with 50 ml portions of water. The solution is dried over anhydrous sodium sulfate and the solvents removed under vacuum. Then, 50 ml toluene is added and removed 3 times under high vacuum to afford as the product 9.2 g crude allyl [(3S 4R, 5R)-3-(1-trichloroethoxycarbonyloxyethyl)-4-[(ethylthio)carbonothioylthio]-2-azetidinone-1-yl]-2-hydroxyacetate.

Rotation: $[\alpha]_D^{26} = +46.9°$ (0.2% in ethanol)

NMR: $\delta = 6.07—6.21$, 1H; $\delta = 4.76$, 2H, s; $\delta = 3.17—3.52$, 3H; $\delta = 1.22—1.54$, 6H.

B. To a solution of 9.0 g crude allyl [(3S, 4R, 5R)-3-(1-trichloroethoxycarbonyloxyethyl)-4-[(ethylthio)carbonothioylthio]-2-azetidinone-1-yl]-2-hydroxyacetate in 125 ml dry methylene chloride at 0°C is added 2.8 g methylsulfonyl chloride followed by 2.5 g triethylamine. The reaction is followed by a thin layer chromatography eluting with 5% ethyl ether/methylene chloride. After stirring 45 minutes, 125 ml methylene chloride is added. The reaction is then washed once with cold 10% phosphoric acid solution, once with water, and once with cold dilute sodium bicarbonate solution and then twice with water. The solution is dried over anhydrous sodium sulfate and the solvents are removed under vacuum. The crude product is chromatographed on coarse silica gel with 20% hexane/chloroform to afford 6.9 g allyl [(3S, 4R, 5R)-3-(1-trichloroethoxycarbonyloxyethyl)-4-[(ethylthio)carbonothioylthio]-2-azetidinone-1-yl]-2-chloroacetate.

IR: 1760—1800 $cm^{-1}$ ($CDCl_3$)

NMR: $\delta = 6.23—6.29$, 1H; $\delta = 4.72$, 2H, s; $\delta = 1.24—1.56$, 6H; ($CDCl_3$).

C. To a solution of 6.9 g allyl [(3S, 4R, 5R)-3-(1-trichloroethoxycarbonyloxyethyl)-4-[(ethylthio)-carbonothioylthio]-2-azetidinone-yl]-2-chloroacetate in 90 ml dimethylformamide at 0°C is added 4.7 g triphenylphosphine. The reaction is allowed to warm to ambient and is stirred 40 hours. (Completion of reaction is determined by thin layer chromatography eluting with methylene chloride). An additional 780 mg triphenylphosphine is added and the reaction stirred at ambient temperature. After 40 hours, the reaction mixture is poured into 300 ml ethyl ether and washed twice with brine solution and 5 times with water. The solvent is dried over anhydrous sodium sulfate and removed under vacuum. The crude product is chromatographed on coarse silica gel with methylene chloride to afford 6.1 g allyl [(3S, 4R, 5R)-3-(1-trichloroethoxycarbonyloxyethyl)-4-[(ethylthio)carbonothioylthio]-2-azetidinone-1-yl]-2-triphenylphosphine acetate.

Rotation: $[\alpha]_D^{26} = +77.0°$

IR: 1760—1780 $cm^{-1}$ (chloroform solution).

NMR: $\delta = 6.3—6.4$, 1H, $\delta = 4.70$, 2H, s; $\delta = 1.16—1.49$, 6H ($CDCl_3$).

D. A solution of 6.1 g allyl [(3S, 4R, 5R)-3-(1-trichloroethoxycarbonyloxyethyl)-4-[(ethylthio)-carbonothioylthio]-2-azetidinone-1-yl]-2-triphenylphosphine acetate in 400 ml toluene is refluxed under nitrogen for 22 hours. (Reaction is followed by thin layer chromatography eluting with 5% ethyl acetate/toluene). The toluene is then removed under high vacuum and the reaction mixture is chromatographed on coarse silica gel with toluene, changing to 10% ethyl acetate/toluene. A mixture of 1.5 g reaction product is isolated which is rechromatographed and then purified by high pressure liquid chromatography with 2% ethyl acetate/toluene to afford 1.18 g allyl (5R, 6S, 8R)-2-ethylthio-6-[1-trichloroethoxycarbonyloxyethyl]penem-3-carboxylate and 240 mg allyl (5,6-)-2-ethylthio-6-[trichloroethoxycarbonyloxyethyl]penem-3-carboxylate.

5R, 6S, 8R Rotation: $[\alpha]_D^{26} = +172.8°$ (0.25% in ethanol).

5,6-cis Rotation: $[\alpha]_D^{26} = +156.4°$ (0.45% in ethanol).

E. To a solution of 1.18 g allyl (5R, 6S, 8R)-2-ethylthio-6-[1-trichloroethoxycarbonyloxyethyl]-penem-3-carboxylate in 9.0 ml tetrahydrofuran under nitrogen is added 3 ml acetic acid and 500 mg activated zinc powder. The reaction is stirred for 2 1/2 hours during which time additional 400 mg zinc metal is added in two portions. The reaction is followed by thin layer chromatography eluting with 5% ethyl acetate/toluene. The reaction mixture is then filtered and 150 ml methylene chloride added. After washing twice with water, 3 times with cold 3% sodium bicarbonate solution and twice with brine solution, the solution is dried over anhydrous sodium sulfate. Removal of the solvents under vacuum affords 720 mg allyl (5R, 6S, 8R)-2-ethylthio-6-(1-hydroxyethyl)-penem-3-carboxylate.

F. To a solution of 700 mg allyl (5R, 6S, 8R)-2-ethylthio-6-(1-hydroxyethyl)penem-3-carboxylate in 4 ml methylene chloride and 8 ml ethyl acetate under nitrogen is added 46.6 mg triphenylphosphine. To this is added 4.86 ml 0.5 molar potassium-2-ethylhexanoate in ethyl acetate. Then, 51.1 mg tetrakis (triphenylphosphine)palladium-(O) is added and the solution is stirred for 15 minutes. An additional 100 mg triphenyl phosphine and 25 mg tetrakis(triphenylphosphine)palladium-(O) is added, followed by 10 ml ethyl ether. The product slowly precipitates and after 1 hour the solution is filtered and washed with ethyl acetate and ethyl ether, to afford 45 mg potassium (5R, 6S, 8R)-2-ethylthio-6-(1-hydroxyethyl)penem-3-carboxylate. To the mother liquor is added 20 ml ethyl ether. After refrigeration overnight, a second crop of crystals is filtered to yield an additional 90 mg of the potassium salt.
NMR: $\delta$ = 1.25—1.49, 6H; $\delta$ = 2.76—3.14, 2H; $\delta$ = 3.85—3.94, 1H; $\delta$ = 4.12—4.37, 1H; $\delta$ = 5.65—5.67, 1H, d; ($D_2O$)
Rotation: $[\alpha]_D^{26} = -145.2°$
IR: 1600 $cm^{-1}$ and 1770 $cm^{-1}$ (nujol).

G. Repetition of the procedures detailed in steps A—F utilizing (3S, 4R, 5R)-*n*-butyl[3-(1-trichloroethoxycarbonyloxyethyl)-(2-azetidinone-4-yl)]trithiocarbonate affords (5R, 6S, 8R)-potassium-6-(1-hydroxyethyl)-2-(*n*-butylthiopenem-3-carboxylate.

## Formulations

The following formulations are to exemplify some of the dosage forms in which the antibacterial agents of this invention may be employed. In each, the active ingredient is designated by the term "Drug" which is meant to indicate:
potassium (5R, 6S, 8R)-(1-hydroxyethyl)-2-ethylthiopenem-3-carboxylate.

It will be appreciated, however, that this compound may be replaced by equally effective quantities of other compounds defined by formula I.

| Injectable Suspension Formulation | mg/ml |
|---|---|
| Sterile drug | 250.0 |
| Benzyl Alcohol | 9.0 |
| Methylparaben | 1.8 |
| Propylparaben | 0.2 |
| Sodium Carboxymethylcellulose | 5.0 |
| Polyethylene Glycol 4000 | 10.0 |
| Povidone | 5.0 |
| Sodium Citrate | 15.0 |
| Disodium Edetate | 0.1 |
| Water for Injection | q.s. |
| To make | 1.0 ml |

Dissolve parabens in a portion of the water for injection by heating it to 65—70°C. Cool to 25—35°C. Charge and dissolve benzyl alcohol, sodium citrate, disodium edetate, PEG 4000, povidone and sodium carboxymethylcellulose. Filter the solution and sterilize by autoclaving. Make a slurry of the sterile active and pass it through a colloid mill. Mix it well with solution from Step 3 and pass it through the mill. Bring the suspension to the final volume/weight and fill into sterile containers.

Capsule Formulation

| Item No. | Ingredient | mg/capsule | mg/capsule |
|---|---|---|---|
| 1 | Drug | 250 | 500 |
| 2 | Lactose, USP | 106 | 123 |
| 3 | Corn Starch, Food Grade | 40 | 70 |
| 4 | Magnesium Stearate, USP | 4 | 7 |
| | | 400 mg | 700 mg |

Mix Item Nos. 1, 2 and 3 in a suitable mixer for 10—15 minutes. Add Item No. 4 and mix for 1—3 minutes. Fill the above mixture into suitable 2-piece hard gelatin capsules.

Tablet Formulation

| Item No. | Ingredient | mg/tablet | mg/tablet |
|---|---|---|---|
| 1 | Drug | 250 | 500 |
| 2 | Lactose, USP | 106 | 112 |
| 3 | Corn Starch, Food Guide as 10% paste in water | 20 | 40 |
| 4 | Corn Starch, Food Guide | 20 | 40 |
| 5 | Magnesium Stearate | 4 | 8 |
| | | 400 mg | 800 mg |

Mix Item Nos. 1 and 2 in a suitable mixer for 10—15 minutes. Granulate the mixture with Item No. 3. Pass the wet granulation through a coarse screen (1/4"). Dry the wet granules for 8—12 hours at 40—50°C. Using a suitable mill, pass the dried granules through a medium screen (No. 12 to No. 16). Add Item No. 4 and mix for 10—15 minutes. Add Item No. 5 and mix further for 1—3 minutes. Compress the mixture to appropriate size and weight on a suitable tablet machine.

**Claims for the Contracting States: BE CH DE FR IT LU NL SE**

1. A compound of the formula

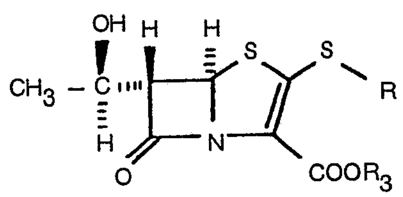

in which R is a $C_1$—$C_6$ alkyl group, and $R_3$ is an alkali metal cation, or a phthalidyl or pivaloyloxymethyl group; or a mixture thereof with its enantiomer.

2. A compound of Claim 1, said compound being sodium or potassium (5R, 6S, 8R)-6-(1-hydroxyethyl)-2-ethylthiopenem-3-carboxylate.

3. A compound of Claim 1, said compound being sodium or potassium (5R, 6S, 8R)-6-(1-hydroxyethyl)-2-methylthiopenem-3-carboxylate.

4. A pharmaceutical composition, comprising a compound as defined in any one of Claims 1 to 3, as an active ingredient.

5. A pharmaceutical composition for oral use, comprising a compound as defined in any one of Claims 1 to 3, as an active ingredient.

6. A pharmaceutical dosage form for oral use comprising a compound as defined in Claim 1, as an active ingredient.

7. A dosage form according to Claim 6, comprising sodium or potassium (5R, 6S, 8R)-6-(1-hydroxyethyl)-2-ethylthiopenem-3-carboxylate.

8. A dosage form according to Claim 6, comprising sodium or potassium (5R, 6S, 8R)-6-(1-hydroxyethyl)-2-methylthiopenem-3-carboxylate.

9. The compounds as defined in Claim 1, for the use in oral treatment of bacterial infections.

10. A process for preparing compounds of the formula

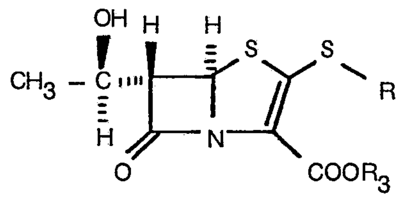

in which R is a $C_1$—$C_6$ alkyl group and $R_3$ is an alkali metal cation, or a phthalidyl or pivaloyloxymethyl group; or a mixture thereof with its enantiomer, which is characterized by cyclising a compound of the formula

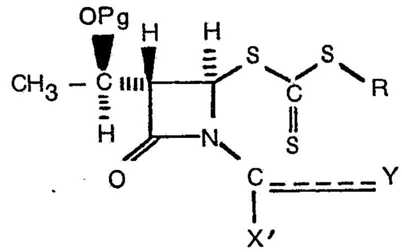

in which X′ is a protected carboxyl group, Pg is a hydroxy protecting group and Y is a phosphonio group being double bonded to the adjacent carbon atom or a phosphonato group with a single bond to the adjacent carbon atom the negative charge of which is compensated by the presence of a cation, or a mixture of stereoisomers containing the same; separating a mixture of diastereoisomers, if a mixture containing diastereoisomers was subjected to cyclisation, before or after removing the protecting groups; and isolating the compound of formula I (in which $R_3$ may be H) or a mixture thereof with its enantiomer as an alkali metal salt, or converting the cyclisation product to the phthalidyl or pivaloyloxymethyl ester or a mixture thereof with its respective enantiomer; if desired the process including the step of separating a mixture of enantiomers if a mixture containing an enantiomer of the compound of formula II was subjected to cyclisation.

11. A process according to Claim 10, in which the group Y in the compound of formula II is a triaryl- or trialkylphosphonio group.

12. A process according to Claim 11, in which Y is triphenylphosphonio.

13. A process according to any one of Claims 10 to 12, in which the cyclisation is carried out at between 30°C. and 160°C. in an inert organic solvent.

14. A process according to any one of Claims 10 to 13, wherein the compound sodium or potassium (5R, 6S, 8R)-6-(1-hydroxy-ethyl)-2-ethylthiopenem-3-carboxylate is produced.

15. A process according to any one of Claims 10 to 13, wherein the compound sodium or potassium (5R, 6S, 8R)-6-(1-hydroxyethyl)-2-methylthiopenem-3-carboxylate is produced.

**Claims for the Contracting State: AT**

1. A process for preparing compounds of the formula

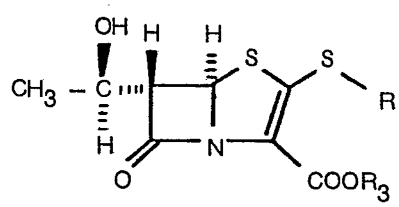

in which R is a $C_1$—$C_6$ alkyl group and $R_3$ is an alkali metal cation, or a phthalidyl or pivaloyloxymethyl group; or a mixture thereof with its enantiomer, which is characterized by cyclising a compound of the formula

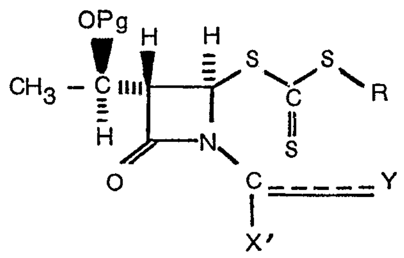

in which X′ is a protected carboxyl group, Pg is a hydroxy protecting group and Y is a phosphonio group being double bonded to the adjacent carbon atom or a phosphonato group with a single bond to the adjacent carbon atom the negative charge of which is compensated by the presence of a cation, or a mixture of stereoisomers containing the same; separating a mixture of diastereoisomers, if a mixture containing diastereoisomers was subjected to cyclisation, before or after removing the protecting groups; and isolating the compound of formula I (in which $R_3$ may be H) or a mixture thereof with its enantiomer as an alkali metal salt, or converting the cyclisation product to the phthalidyl or pivaloyloxymethyl ester or a mixture thereof with its respective enantiomer; if desired the process including the step of separating a mixture of enantiomers if a mixture containing an enantiomer of the compound of formula II was subjected to cyclisation.

2. A process according to Claim 1, in which the group Y in the compound of formula II is a triaryl- or trialkylphosphonio group.

3. A process according to Claim 2, in which Y is triphenylphosphonio.

4. A process according to any one of Claims 1 to 3, in which the cyclisation is carried out at between 30°C and 160°C. in an inert organic solvent.

5. A process according to any one of Claims 1 to 4, wherein the compound sodium or potassium (5R, 6S, 8R)-6-(1-hydroxy-ethyl)-2-ethylthiopenem-3-carboxylate is produced.

6. A process according to any one of Claims 1 to 4, wherein the compound sodium or potassium (5R, 6S, 8R)-6-(1-hydroxyethyl)-2-methylthiopenem-3-carboxylate is produced.

7. A process for preparing a pharmaceutical composition which comprises mixing a compound of formula I as defined in Claim 1, with a pharmaceutical carrier or excipient.

8. A process for preparing a pharmaceutical composition for oral use which comprises mixing a compound of formula I as defined in Claim 1, with a pharmaceutical carrier or excipient.

9. A process for preparing a pharmaceutical dosage form for oral use which comprises mixing a compound of formula I as defined in Claim 1, with a pharmaceutical carrier or excipient.

10. A process for preparing a pharmaceutical dosage form for oral use which comprises mixing a compound as defined in Claim 5, with a pharmaceutical carrier or excipient.

11. A process for preparing a pharmaceutical dosage form for oral use which comprises mixing a compound as defined in Claim 6, with a pharmaceutical carrier or excipient.

**Revendications pour les Etats contractants: BE CH DE FR IT LU NL SE**

1. Composé de formule

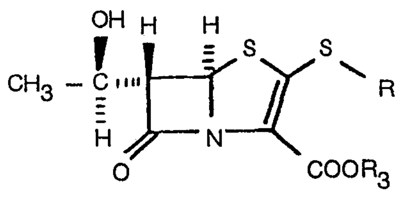

où R est un groupe alcoyle de 1 à 6 atomes de carbone et $R_3$ est un cation d'un métal alcalin ou un groupe phtalidyle ou pivaloyloxyméthyle; ou son mélange avec son énantiomère.

2. Composé selon la revendication 1, ledit composé étant sodium ou potassium (5R, 6S, 8R)-6-(1-hydroxyéthyl)-2-éthylthiopenem-3-carboxylate.

3. Composé selon la revendication 1, ledit composé étant sodium ou potassium (5R, 6S, 8R)-6-(1-hydroxyéthyl)-2-méthylthiopenem-3-carboxylate.

4. Composition pharmaceutique comprenant un composé tel que défini selon l'une des revendications 1 à 3, comme ingrédient actif.

5. Composition pharmaceutique pour un usage oral, comprenant un composé tel que défini selon l'une des revendications 1 à 3, comme ingrédient actif.

6. Forme de dosage pharmaceutique pour un usage oral comprenant un composé selon la revendication 1, comme ingrédient actif.

7. Forme de dosage selon la revendication 6, comprenant du sodium ou potassium (5R, 6S, 8R)-6-(1-hydroxyéthyl)-2-éthylthiopenem-3-carboxylate.

8. Forme de dosage selon la revendication 6, comprenant du sodium ou potassium (5R, 6S, 8R)-6-(1-hydroxyéthyl)-2-méthylthiopenem-3-carboxylate.

9. Composé selon la revendication 1 pour l'utilisation dans le traitement oral des infections bactériennes.

10. Procédé de préparation de composés de formule

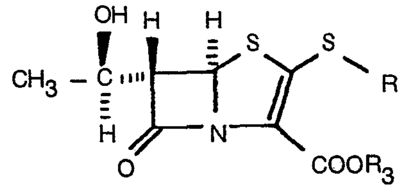

où R est un groupe alcoyle de 1 à 6 atomes de carbone et $R_3$ est un cation d'un métal alcalin, ou un groupe phtalidyle ou pivaloyloxyméthyle; ou son mélange avec son énantiomère, caractérisé par la cyclisation d'un composé de formule

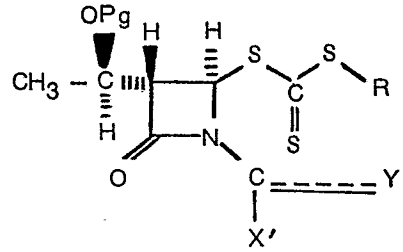

où X' est un groupe carboxyle protégé, Pg est un groupe hydroxy protecteur et Y est un groupe phosphonio qui est doublement lié à l'atome de carbone adjacent ou un groupe phosphonato avec une simple liaison à l'atome de carbone adjacent, dont la charge négative est compensée par la présence d'un cation, ou un mélange de stéréoisomères le contenant; la séparation d'un mélange de diastéréoisomères, si un mélange contenant, des diastéréoisomères a été soumis à une cyclisation, avant ou après enlèvement des groupes protecteurs; et l'isolement du composé de formule I (où $R_3$ peut être H) ou son mélange avec son énantiomère sous forme d'un sel d'un métal alcalin, ou la conversion du produit de cyclisation en phtalidyl ou pivaloyloxyméthyl ester ou son mélange avec son énantiomère respectif; si on le souhaite, le procédé comprend l'étape de séparer un mélange d'énantiomères si un mélange contenant un énantiomère du composé de formule II a été soumis à une cyclisation.

11. Procédé selon la revendication 10 où le groupe Y dans le composé de formule II est un groupe triaryl- ou trialkylphosphonio.

12. Procédé selon la revendication 11 où Y est triphénylphosphonio.

13. Procédé selon l'une des revendications 10 à 12 où la cyclisation est effectuée entre 30 et 160°C dans un solvant organique inerte.

14. Procédé selon l'une des revendications 10 à 13 où le composé sodium ou potassium (5R, 6S, 8R)-6-(1-hydroxyéthyl)-2-éthylthiopenem-3-carboxylate est produit.

15. Procédé selon l'une des revendications 10 à 13 où le composé sodium ou potassium (5R, 6S, 8R)-6-(1-hydroxyéthyl)-2-méthylthiopenem-3-carboxylate est produit.

**Revendications pour l'Etat contractant: AT**

1. Procédé de préparation de composés de formule

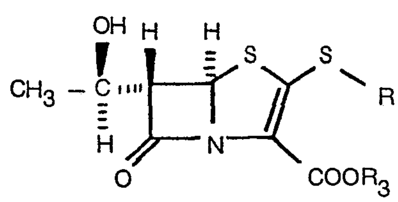

où R est un groupe alcoyle de 1 à 6 atomes de carbone et $R_3$ est un cation d'un métal alcalin, ou un groupe phtalidyle ou pivaloyloxyméthyle; ou son mélange avec son énantiomère, caractérisé par la cyclisation d'un composé de formule

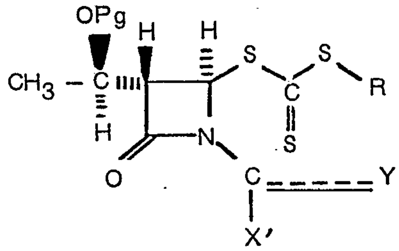

où X′ est un groupe carboxyle protégé, Pg est un groupe hydroxy protecteur et Y est un groupe phosphonio qui est doublement lié à l'atome de carbone adjacent ou un groupe phosphonato avec une simple liaison à l'atome de carbone adjacent, dont la charge négative est compensée par la présence d'un cation, ou un mélange de stéréoisomères le contenant; la séparation d'un mélange de diastéréoisomères, si un mélange contenant des diastéréoisomères a été soumis à une cyclisation, avant ou après enlèvement des groupes protecteurs; et l'isolement du composé de formule I (où $R_3$ peut être H) ou son mélange avec son énantiomère sous forme d'un sel d'un métal alcalin, ou la conversion du produit de cyclisation en phtalidyl ou pivaloyloxyméthyl ester ou son mélange avec son énantiomère respectif; si on le souhaite, le procédé comprend l'étape de séparer un mélange d'énantiomères si un mélange contenant un énantiomère du composé de formule II a été soumis à une cyclisation.

2. Procédé selon la revendication 10 où le groupe Y dans le composé de formule II est un groupe triaryl- ou trialkylphosphonio.

3. Procédé selon la revendication 11 où Y est triphénylphosphonio.

4. Procédé selon l'une des revendications 10 à 12 où la cyclisation est effectuée entre 30 et 160°C dans un solvant organique inerte.

5. Procédé selon l'une des revendications 10 à 13 où le composé sodium ou potassium (5R, 6S, 8R)-6-(1-hydroxyéthyl)-2-éthylthiopenem-3-carboxylate est produit.

6. Procédé selon l'une des revendications 10 à 13 où le composé sodium ou potassium (5R, 6S, 8R)-6-(1-hydroxyéthyl)-2-méthylthiopenem-3-carboxylate est produit.

7. Procédé de préparation d'une composition pharmaceutique qui consiste à mélanger un composé de formule I tel que défini à la revendication 1 avec un véhicule ou excipient pharmaceutique.

8. Procédé de préparation d'une composition pharmaceutique pour un usage oral qui consiste à mélanger un composé de formule I tel que défini à la revendication 1 avec un véhicule ou excipient pharmaceutique.

9. Procédé de préparation d'une forme de dosage pharmaceutique pour un usage oral qui consiste à mélanger un composé de formule I tel que défini à la revendication 1 avec un véhicule ou excipient pharmaceutique.

10. Procédé de préparation d'une forme de dosage pharmaceutique pour un usage oral qui consiste à mélanger un composé selon la revendication 5 avec un véhicule ou excipient pharmaceutique.

11. Procédé de préparation d'une forme de dosage pharmaceutique pour un usage oral qui consiste à mélanger un composé tel que défini à la revendication 6, avec un véhicule ou excipient pharmaceutique.

**Patentansprüche für die Vertragsstaaten: BE CH DE FR IT LU NL SE**

1. Verbindung der Formel

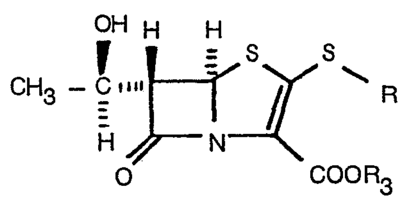

(I)

in der R eine $C_1$- bis $C_6$-Alkyl-Gruppe ist und $R_3$ ein Alkalimetall-Kation oder eine Phthalidyl- oder Pivaloyloxymethyl-Gruppe ist, oder eine Mischung derselben mit ihrem Enantiomer.

2. Verbindung Natrium- oder Kalium (5R, 6S, 8R)-6-(1-hydroxyäthyl)-2-äthylthiopenem-3-carboxylat nach Anspruch 1.

3. Verbindung Natrium- oder Kalium (5R, 6S, 8R)-6-(1-hydroxyäthyl)-2-methylthiopenem-3-carboxylat nach Anspruch 1.

4. Pharmazeutisch Zusammensetzung, enthaltend als einen Wirkstoff eine der in einem der Ansprüche 1 bis 3 definierten Verbindungen.

5. Pharmazeutisch Zusammensetzung zur oralen Verabreichung, enthaltend als einen Wirkstoff eine der in einem der Ansprüche 1 bis 3 definierten Verbindungen.

6. Pharmazeutische Dosierungsform zur oralen Verabreichung, enthaltend als einen Wirkstoff eine der in Anspruch 1 definierten Verbindungen.

7. Dosierungsform nach Anspruch 6, enthaltend Natrium- oder Kalium (5R, 6S, 8R)-6-(1-hydroxyäthyl)-2-äthylthiopenem-3-carboxylat.

8. Dosierungsform nach Anspruch 6, enthaltend Natrium- oder Kalium (5R, 6S, 8R)-6-(1-hydroxyäthyl)-2-methylthiopenem-3-carboxylat.

9. Verbindungen nach Anspruch 1 zur Verwendung bei der oralen Behandlung bakterieller Infektionen.

10. Verfahren zur Herstellung von Verbindungen der Formel

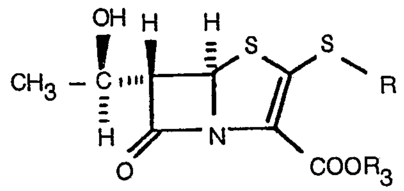

(I)

in der R eine $C_1$- bis $C_6$-Alkyl-Gruppe ist und $R_3$ ein Alkalimetall-Kation oder eine Phthalidyl- oder Pivaloyloxymethyl-Gruppe ist oder einer Mischung derselben mit ihrem Enantiomer, dadurch gekennzeichnet, daß eine Verbindung der Formel

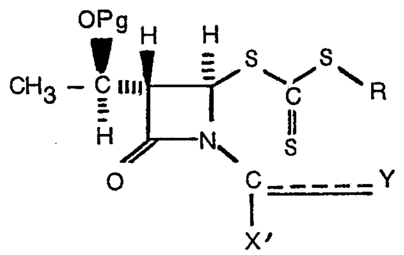

(II)

in der

X′ eine geschützte Carboxyl-Gruppe ist,

Pg eine Hydroxyl-Schutzgruppe ist und

Y eine an das benachbarte Kohlenstoff-Atom doppelt gebundene Phosphoniogruppe oder eine Phosphonatgruppe mit einer Einfachbindung an das benachbarte Kohlenstoff-Atom ist, deren negative Ladung durch die Anwesenheit eines Kations ausgeglichen ist, oder eine dieselbe enthaltende Mischung von Stereoisomeren cyclisiert wird, ein Gemisch der Diastereomeren, wenn eine Diastereomere enthaltende Mischung der Cyclisierung unterworfen wurde, vor oder nach dem Entfernen der Schutzgruppen getrennt wird und die Verbindung der Formel I (in der $R_3$ H sein kann) oder eine Mischung derselben mit ihrem Enantiomer als Alkalimetall-Salz isoliert wird oder das Cyclisierungsprodukt in den Phthalidyl- oder Pivaloyloxymethylester oder eine Mischung desselben mit seinem entsprechenden Enantiomer umgewandelt wird, wobei, falls erwünscht, das Verfahren den Schritt der Trennung eines Enantiomeren-Gemischs einschließt, falls eine ein Enantiomer der Verbindung der Formel II enthaltende Mischung der Cyclisierung unterworfen wurde.

11. Verfahren nach Anspruch 10, dadurch gekennzeichnet, daß die Gruppe Y in der Verbindung der Formel II eine Triaryl- oder Trialkylphosphonio-Gruppe ist.

12. Verfahren nach Anspruch 11, dadurch gekennzeichnet, daß Y Triphenylphosphonio ist.

13. Verfahren nach einem der Ansprüche 10 bis 12, dadurch gekennzeichnet, daß die Cyclisierung zwischen 30°C und 160°C in einem inerten organischen Lösungsmittel durchgeführt wird.

14. Verfahren nach einem der Ansprüche 10 bis 13, dadurch gekennzeichnet, daß die Verbindung Natrium- oder Kalium (5R, 6S, 8R)-6-(1-hydroxyäthyl)-2-äthylthiopenem-3-carboxylat hergestellt wird.

15. Verfahren nach einem der Ansprüche 10 bis 13, dadurch gekennzeichnet, daß die Verbindung Natrium- oder Kalium (5R, 6S, 8R)-6-(1-hydroxyäthyl)-2-methylthiopenem-3-carboxylat hergestellt wird.

**Patentansprüche für den Vertragsstaat: AT**

1. Verfahren zur Herstellung von Verbindungen der Formel

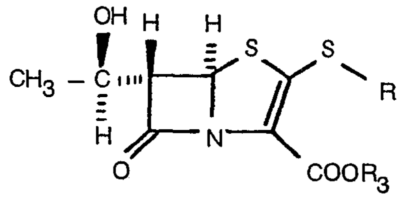

(I)

in der R eine $C_1$- bis $C_6$-Alkyl-Gruppe ist und $R_3$ ein Alkalimetall-Kation oder eine Phthalidyl- oder Pivaloyloxymethyl-Gruppe ist oder einer Mischung derselben mit ihrem Enantiomer, dadurch gekennzeichnet, daß eine Verbindung der Formel

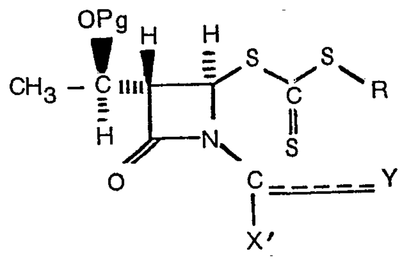

(II)

in der

X′ eine geschützte Carboxyl-Gruppe ist,

Pg eine Hydroxyl-Schutzgruppe ist und

Y eine an das benachbarte Kohlenstoff-Atom doppelt gebundene Phosphoniogruppe oder eine Phosphonatgruppe mit einer Einfachbindung an das benachbarte Kohlenstoff-Atom ist, deren negative Ladung durch die Anwesenheit eines Kations ausgeglichen ist, oder eine dieselbe enthaltende Mischung von Stereoisomeren cyclisiert wird, ein Gemisch der Diastereomeren, wenn eine Diastereomere enthaltende Mischung der Cyclisierung unterworfen wurde, vor oder nach dem Entfernen der Schutzgruppen getrennt wird und die Verbindung der Formel I (in der $R_3$ H sein kann) oder eine Mischung derselben mit ihrem Enantiomer als Alkalimetall-Salz isoliert wird oder das Cyclisierungsprodukt in den Phthalidyl- oder Pivaloyloxymethylester oder eine Mischung desselben mit seinem entsprechenden Enantiomer umgewandelt wird, wobei, falls erwünscht, das Verfahren den Schritt der Trennung eines Enantiomeren-Gemischs einschließt, falls eine ein Enantiomer der Verbindung der Formel II enthaltende Mischung der Cyclisierung unterworfen wurde.

2. Verfahren nach Anspruch 1, dadurch gekennzeichnet, daß die Gruppe Y in der Verbindung der Formel II eine Triaryl- oder Trialkylphosphonio-Gruppe ist.

3. Verfahren nach Anspruch 2, dadurch gekennzeichnet, daß Y Triphenylphosphonio ist.

4. Verfahren nach einem der Ansprüche 1 bis 3, dadurch gekennzeichnet, daß die Cyclisierung zwischen 30°C und 160°C in einem inerten organischen Lösungsmittel durchgeführt wird.

5. Verfahren nach einem der Ansprüche 1 bis 4, dadurch gekennzeichnet, daß die Verbindung Natrium- oder Kalium (5R, 6S, 8R)-6-(1-hydroxyäthyl)-2-äthylthiopenem-3-carboxylat hergestellt wird.

6. Verfahren nach einem der Ansprüche 1 bis 4, dadurch gekennzeichnet, daß die Verbindung Natrium- oder Kalium (5R, 6S, 8R)-6-(1-hydroxyäthyl)-2-methylthiopenem-3-carboxylat hergestellt wird.

7. Verfahren zur Herstellung einer pharmazeutischen Zusammensetzung, dadurch gekennzeichnet, daß eine Verbindung der in Anspruch 1 definierten Formel I mit einem pharmazeutischen Träger oder Hilfsstoff vermischt wird.

8. Verfahren zur Herstellung einer pharmazeutischen Zusammensetzung zur oralen Verabreichung, dadurch gekennzeichnet, daß eine Verbindung der in Anspruch 1 definierten Formel I mit einem pharmazeutischen Träger oder Hilfsstoff vermischt wird.

9. Verfahren zur Herstellung einer pharmazeutischen Dosierungsform zur oralen Verabreichung, dadurch gekennzeichnet, daß eine Verbindung der in Anspruch 1 definierten Formel I mit einem pharmazeutischen Träger oder Hilfsstoff vermischt wird.

10. Verfahren zur Herstellung einer pharmazeutischen Dosierungsform zur oralen Verabreichung, dadurch gekennzeichnet, daß eine in Anspruch 5 definierte Verbindung mit einem pharmazeutischen Träger oder Hilfsstoff vermischt wird.

11. Verfahren zur Herstellung einer pharmazeutischen Dosierungsform zur oralen Verabreichung, dadurch gekennzeichnet, daß eine in Anspruch 6 definierte Verbindung mit einem pharmazeutischen Träger oder Hilfsstoff vermischt wird.